# EUROPÄISCHE PATENTANMELDUNG

(11) **EP 0 540 461 A1**
(43) Veröffentlichungstag der Anmeldung: **05.05.1993**
(21) Anmeldenummer: 92810738.2
(22) Anmeldetag: 01.10.1992
(51) Int. Cl.: A61B 19/08, A61B 17/34, A61B 8/08

(54) **Steriles Punktiergerät für Blutgefässe mit nichtsteriler Ultraschallsonde und Vorrichtung zum Vorbereiten des Geräts**

(30) Priorität: 29.10.1991 CH 3157/91
(71) Anmelder: SULZER Medizinaltechnik AG, CH-8404 Winterthur (CH)
(72) Erfinder: Dardel, Erich, Dr. med., CH-8472 Seuzach (CH)
(74) Vertreter: Hammer, Bruno, Dr.

(57) **Zusammenfassung**

Das sterile Punktiergerät für Blutgefässe weist eine Ultraschallsonde (1) auf, die nichtsteril verwendbar ist. Mittels der erfindungsgemässen Vorrichtung lässt sich die Sonde (1) in eine Kapsel (12, 13) des Punktiergeräts einschleusen. Die Vorrichtung besteht aus einem dichten Beutel (16) mit sterilem Inhalt (10), wobei im Beutel ein Halter (11) mit der Kapsel für die Ultraschallsonde enthalten ist und eine schleusenartige Eintrittsstelle, die mit der geöffneten Kapsel in Verbindung steht, an der Beutelwand angeordnet ist. Die Vorrichtung ist als Einwegartikel vorgesehen. Die Ultraschallsonde, die mehrfach verwendbar ist, wird kurz vor dem Punktieren in das Gerät eingeschleust.

## Beschreibung

Die Erfindung bezieht sich auf eine Vorrichtung zum Vorbereiten eines sterilen Punktiergeräts für Blutgefässe, bei dem eine nichtsterile Ultraschallsonde verwendbar ist, sowie auf ein solches Punktiergerät.

Die Ultraschallsonde eines solchen Punktiergeräts weist eine Sender/Empfänger-Anordnung auf, mit der Ultraschall in den Körper eingestrahlt und ein Signal des teilweise reflektierten Strahls registriert werden kann. Durch Reflexion am strömenden Blut ergibt sich dank des Dopplereffekts eine Fequenzverschiebung, aus der sich ein akustisches und/oder optisches Signal gewinnen lässt. Durch Suchen eines Maximums eines solchen Signals kann die Einstichstelle für die Punktiernadel gefunden werden.

Derartige Geräte sind beispielsweise aus der CH-PS 676 787 bekannt. Die Sender/Empfänger-Anordnung weist eine Konstruktion auf, bei der ein Führungskanal für die Nadel durch den Schallschwinger verläuft. Diese Konstruktion ist nachteilig, da das Punktieren unter sterilen Bedingungen durchgeführt werden muss und da eine notwendige Gassterilisation der Ultraschallsonde sehr zeitaufwendig ist. Bei einer einfacheren Ultraschallsonde, die keinen integrierten Führungskanal aufweist, lässt sich die Sterilität durch eine sterile, zum einmaligen Gebrauch bestimmte Hülle erreichen (siehe beispielsweise EP-A 0 104 618). Bei diesen einfacheren Sonden kann allerdings die Nadel nicht koaxial zum emittierten Ultraschallstrahl geführt werden.

Es ist Aufgabe der Erfindung, einerseits ein Punktiergerät, bei dem eine nichtsterile Ultraschallsonde verwendbar ist, zu schaffen und andererseits eine Vorrichtung zu schaffen, mit der das Punktiergerät für den Einsatz bei sterilen Bedingungen vorbereitet werden kann. Diese Aufgabe wird durch die Merkmale der unabhängigen Ansprüche 1 bzw. 4 gelöst. Ausserdem soll bei diesem erfindungsgemässen Punktiergerät eine zur Richtung des emittierten Strahls zumindest angenähert koaxiale Nadelführung möglich sein.

Das erfindungsgemässe Punktiergerät besteht neben den in Anspruch 1 genannten Kapsel und Halter auch aus einem stationären Geräteteil mit beispielsweise optischen und/oder akustischen Anzeigemitteln sowie einem Verbindungskabel für die Energieversorgung der Ultraschallsonde und für die Signalübertragung. Das stationäre Geräteteil braucht nicht steril zu sein, wenn es einen genügend grossen Abstand zum Behandlungsbereich aufweist; es kann allerdings auch von einem sterilen Sack umhüllt sein. Das Verbindungskabel, das am stationären Geräteteil einsteckbar ist, muss steril sein; es gehört auch zum Beutelinhalt der erfindungsgemässen Vorrichtung. Die nichtsterile Ultraschallsonde wird kurz vor dem Punktieren eingeschleust. Anschliessend wird der Beutel geöffnet - beispielsweise aufgerissen - und der Inhalt dem Arzt steril übergeben.

Es weiden verschiedene Ausführungsbeispiele des erfindungsgemässen Punktiergeräts beschrieben. Bei einem wird eine zylindrische Ultraschallsonde verwendet. An der Kapsel ist ein Koppelstück für den in der Sonde erzeugten sowie im Körper reflektierten Ultraschallstrahl angeordnet. In diesem Koppelstück befinden sich ein Spiegel für die Umlenkung des Ultraschalls und eine Führung für eine Punktiernadel. Die Führung ist derart angelegt, dass die Punktiernadel zumindest angenähert koaxial zur Richtung des emittierten Strahls bewegbar ist. Bei einem zweiten Ausführungsbeispiel besteht die Ultraschallsonde aus zwei über ein Scharnier verbundenen Zylinderhälften. In der einen Hälfte befindet sich der Sender und in der anderen der Empfänger der Ultraschallsonde (vgl. z.B. US-PS 3556079). In der Kapsel ist eine rohrförmige Führung für die Punktiernadel angeordnet, die entlang der Längsachse der eingesetzten, zusammengeklappten Ultraschallsonde verläuft und die nebst der Führungsfunktion für eine Abschirmung der Punktiernadel gegen die nichtsterile Sonde dient. Die Ultraschallsonde kann auch aus einem Zylinder mit Nut bestehen, wobei die Nadelführung in die Nut einschiebbar ist. Bei einer weiteren Ausführungsform weist die zylindrische Sonde anstelle der Nut einen axialen, seitlich geschlossenen Kanal auf. Bezüglich der Ultraschallortung lässt sich noch anmerken, dass selbsverständlich auch nur ein gepulst betriebener Schwingkristall vorgesehen sein kann, der sowohl als Sender als auch als Empfänger der Ultraschallsonde funktioniert.

Die erfindungsgemässe Vorrichtung sowie das zugeordnete Sondenteil, bestehend aus Kapsel, Halter und Verbindungskabel aber ohne Ultraschallsonde, sind als Einwegartikel vorgesehen. Als Herstellungsmaterial wird vorzugsweise Kunststoff verwendet, wobei aber auch einzelne Komponenten wie beispielsweise der Ultraschallspiegel aus Metall oder anderen Werkstoffen hergestellt werden können.

Nachfolgend wird die Erfindung anhand der Figuren näher erläutert. Es zeigen:
- Fig. 1a: eine Ultraschallsonde, die aus zwei über ein Scharnier verbundene Zylinderhälften besteht,
- Fig. 1b: eine Ultraschallsonde in Form eines Zylinders mit Nut,
- Fig. 2: das Sondenteil eines erfindungsgemässen Punktiergeräts für die Ultraschallsonde der Fig. 1a oder 1b,
- Fig. 3: einen Teil einer erfindungsgemässen Vorrichtung zum Vorbereiten des Punktiergeräts der Fig.2,
- Fig.4a-c: drei Phasen beim Einschleusen der Ultraschallsonde mittels der Vorrichtung der Fig.3,
- Fig. 5: eine zylindrische Ultraschallsonde,
- Fig. 6: ein Sondenteil für die Ultraschallsonde der Fig.5,
- Fig. 7: Teile der schleusenartigen Eintrittsstelle der erfindungsgemässen Vorrichtung,
- Fig. 8: ein Scharnier mit schnappververbindungsartigen Gelenken,
- Fig. 9: eine variante Ausführung der Vorrichtung gemäss Fig.3 und
- Fig.10a-c: eine weitere variante Ausführungsform.

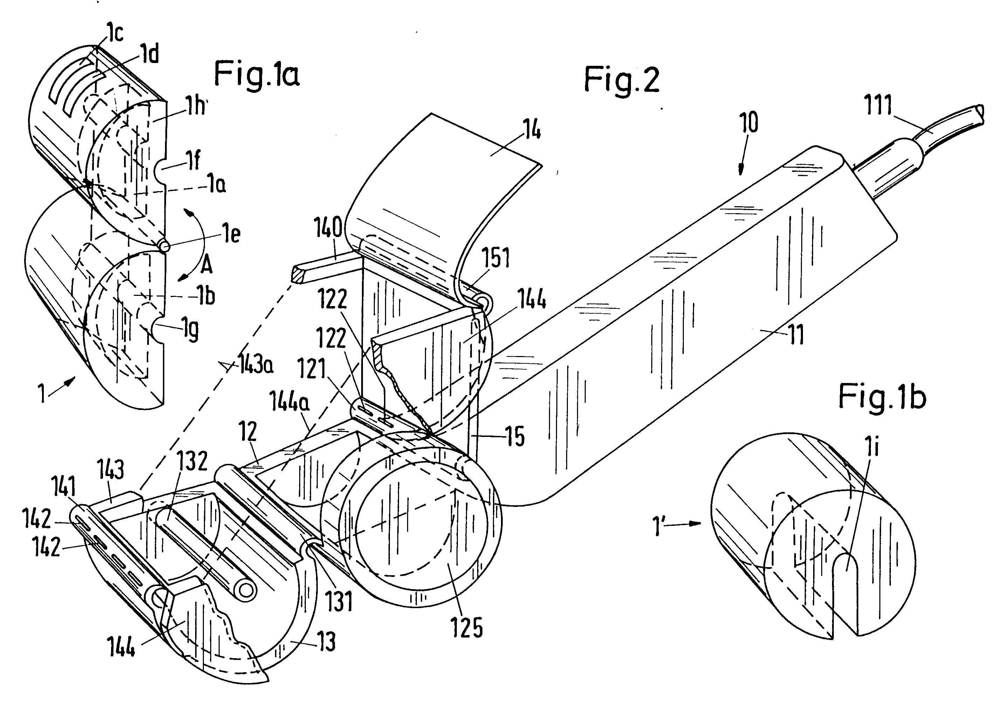

Die Ultraschallsonde 1 der Fig.1a enthält einen Sender 1a und einen Empfänger 1b, die mit den beiden Kontaktstellen 1c und 1d an der Sondenobërfläche verbunden sind. Die beiden Hälften der Sonde 1 sind um das Scharnier 1e zu einem Kreiszylinder gemäss Doppelpfeil A zusammenklappbar. Im zusammengeklappten Zustand weist die Sonde 1 einen Kanal für die Nadelführung auf, der durch die beiden Teile 1f und 1g gebildet wird. Der im Sender 1a erzeugte Ultraschallstrahl wird über die Stirnfläche 1h emittiert. Fig.1b zeigt eine alternative Ultraschallsonde 1′ mit einer Nut 1i, mittels welcher die Sonde 1′ über die Nadelführung schiebbar ist. Die (nicht dargestellten) Kontaktstellen - entsprechend den Kontaktstellen 1c und 1d - können an den inneren Seitenflächen der Nut 1i angeordnet sein.

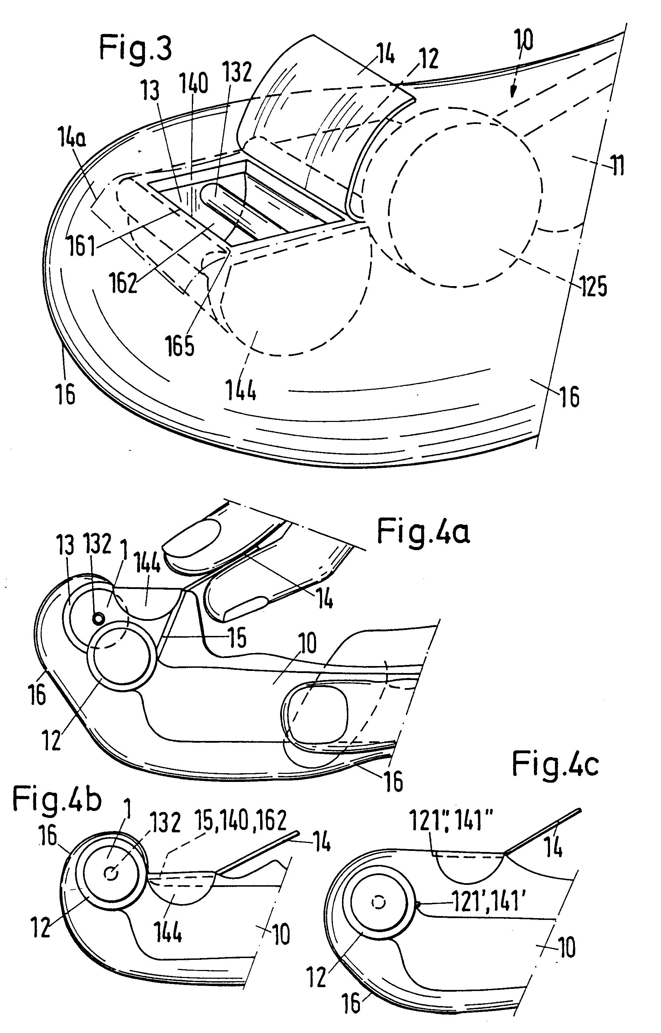

Das Sondenteil 10 der Fig.2 besteht aus dem als Griff dienenden Halter 11, dem Verbindungskabel 111, von dem nur ein kurzes Stück dargestellt ist, und der aus den beiden Schalenteile 12 und 13 bestehenden Kapsel für die Ultraschallsonde 1 bzw. 1′. Die beiden Schalenteilen 12 und 13 sind durch ein Scharnier 131 miteinander verbunden. Durch zwei weitere Scharniere 121 und 141 ist die Kapsel mit Komponenten der - nicht vollständig dargestellten - erfindungsgemässen Vorrichtung verbunden, welche Teile der schleusenartigen Eintrittsstelle sind. In Fig.3 ist gezeigt, wie das Sondenteil 10 im Beutel 16 der erfindungsgemässen Vorrichtung angeordnet ist.

Die erwähnten Komponenten der Eintrittsstelle oder Einschleusvorrichtung sind der erste Deckel 14 auf dem Rahmen 140 und der zweite Deckel 15, der mit dem Rahmen 140 durch ein Scharnier 151 verbunden ist. Der Rahmen verstärkt den Rand 161 der Beutelöffnung 162. Der erste Deckel 14 ist am Rahmen 140 längs des Scharniers 151 befestigt; er verschliesst vor dem Einschleusen der Ultraschallsonde die Beutelöffnung 162 dicht, ist aber lösbar mit dem Rand 161 verbunden. (In Fig.3 ist der Deckelrand 14a beim versiegelten Beutel 16 strichpunktiert angedeutet.) Der Rahmen wird senkrecht zu den beiden Scharnieren 141 und 151 durch den Steg 143 und den oberen Rand des Schilds 144 gebildet. (Diese beiden Komponenten 143 und 144 sind in Fig.2 aus Darstellungsgründen je in zwei Teile zerbrochen gezeichnet; man muss sie sich entlang den gestrichelten Linien 143a und 144a zusammengefügt denken.) Die Scharniere 121, 131, 141 und 151 werden beispielsweise als "Filmscharniere" ausgebildet, wie es in Fig.2 angedeutet ist.

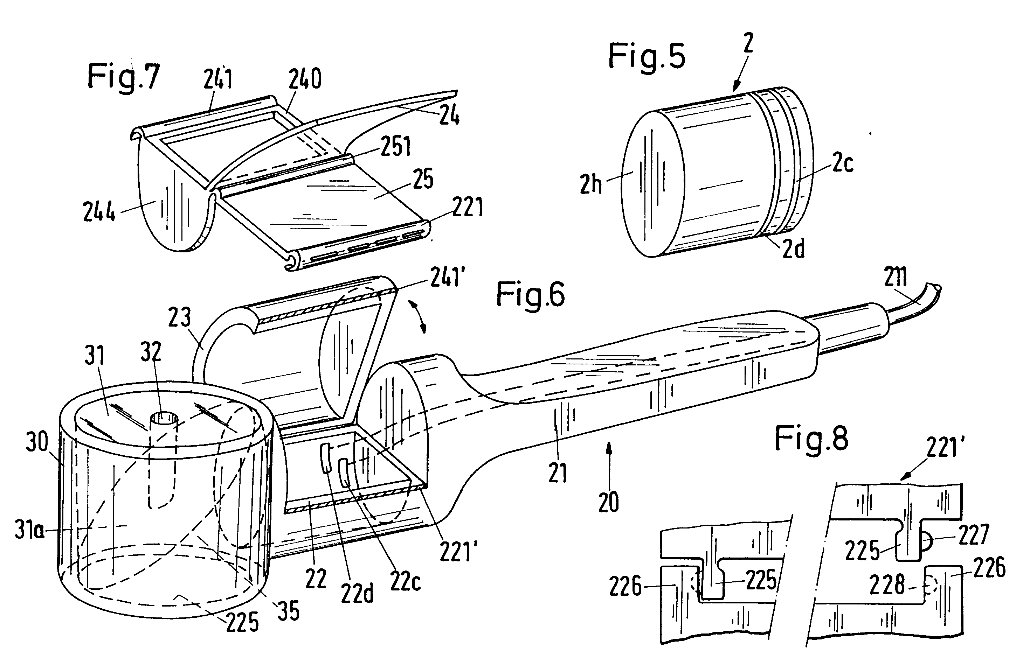

In den Figuren 2 und 3 ist der erste Deckel 14 von der Beutelöffnung 162 abgezogen; die Ultraschallsonde 1 kann in das Schalenteil 13 eingelegt werden. Der Schild 144 deckt das stirnseitig offene Schalenteil 13 ab und verhindert so bei offenem Beutel 16 einen direkten Durchgang in das sterile Beutelinnere. Nach dem Einlegen umschliesst die Ultraschallsonde 1 in zusammengeklapptem Zustand die Nadelführung 132. Durch Ziehen am Deckel 14 - wie es in Fig.4a gezeigt ist - kann nun das Schalenteil 13 mit der eingelegten Sonde 1 auf das andere Schalenteil 14 der Kapsel verschwenkt werden, wobei gleichzeitig auch der zweite Deckel 15 und die übrigen Teile der Eintrittsstelle mitverschwenkt werden. In Fig.4b befindet sich die Sonde 1 vollständig in der Kapsel eingeschlossen und die Beutelöffnung 162 ist von innen durch den zweiten Deckel 15 verschlossen. Schliesslich wird das Sondenteil 1 von der Eintrittsstelle getrennt (Fig.4c) wobei die Scharniere 121 und 141 je in zwei Teile 121′ und 121˝ bzw. 141′ und 141˝ zerrissen werden. Perforationen 122 und 142 in den Scharnieren 121 bzw. 141 (siehe Fig.2) können das Abtrennen erleichtern. Fig.8 zeigt eine variante Form eines Scharniers 121′ mit schnappverschlussartigen Gelenken, die aus den beiden Teilen 225 und 226 bestehen. Eine kugelförmige Erhebung 227 wird von einer entsprechenden Vertiefung 228 aufgenommen. Links in Fig.8 sind die Gelenkteile 225 und 226 zusammengefügt, rechts getrennt. Solche Scharniere haben gegenüber Filmscharnieren den Vorteil, dass sie sich leicht trennen lassen.

Der Rand der Beutelöffnung 165 kann beispielsweise mit einem Haftmittel für den ersten Deckel 14 beschichtet werden, um eine lösbare Versiegelung des Beutels 16 zu erhalten. Wird ein thermoplastischer Kunststoff als Material für den Beutel und/oder den Deckel verwendet, so kann die Versiegelung auch mittels einer Wärmeeinwirkung erreicht werden. Auch der Rahmen 140 kann auf der Innenseite an den Kontaktstellen zum zweiten Deckel 15 mit einem Haftmittel beschichtet werden. Ferner können, um einen sicheren Einschluss der Ultraschallsonde 1 in der Kapsel zu gewährleisten, auch die Kontaktflächen zwischen den Schalenteilen 12 und 13, welche bei geschlossener Kapsel aufeinander zu liegen kommen, zumindest teilweise mit einem Haftmittel versehen werden. Anstelle des Haftmittels kann auch eine Schnappverbindung oder Schliesszapfen den gleichen Zweck erfüllen.

Beim Punktieren wird das Sondenteil 10 mit der Kapselwand 125 auf den Körper aufgelegt. Diese Kapselwand ist aus einem für die Punktiernadel durchstechbaren Material hergestellt, beispielsweise aus einem elastomeren Kunststoff oder aus Gelatine.

Anstelle der etwas komplizierten Ultraschallsonde 1 kann auch eine einfache zylindrische Sonde 2 (Fig.5) verwendet werden. Allerdings muss dann das Sondenteil 20 (Fig.6) etwas aufwendiger gestaltet werden. Die Vorrichtung zum Vorbereiten des Punktiergeräts sowie die Kapselung der Ultraschallsonde können im wesentlich unverändert vom ersten Ausführungsbeispiel übernommen werden. Die Kapsel besteht aus den beiden Schalenteilen 22 und 23, die an den Schnittflächen 221′ und 241′ über die Scharnier 221 bzw. 241 mit den übrigen Komponenten der Eintrittstelle (Fig.7) verbunden sind. Diese Komponenten sind wiederum ein erster Deckel 24, ein Rahmen 240, ein zweiter Deckel 25 und ein Schild 244. Der Rahmen 240 verstärkt wie beim ersten Ausführungsbeispiel die Öffnung eines nicht dargestellten Beutels. Im Schalenteil 22 der Kapsel, der starr mit dem Halter 21 verbunden ist, sind die elektrischen Kontaktstellen 22c und 22d angeordnet, über die der Anschluss der Ultraschallsonde 2 - über die Verbindungsleitung 211 - an ein nicht dargestelltes stationäres Geräteteil herstellbar ist. Die entsprechenden Kontaktstellen 2c und 2d an der Sonde 2 sind beispielsweise ringförmig ausgeführt, sodass beim Einschleusen nicht auf eine besondere Stellung der Sonde geachtet werden muss.

Die Ultraschallsonde 2 ist mit ihrer abstrahlenden Stirnfläche 2h im Sondenteil 20 gegen das Koppelstück 30 gerichtet. In diesem Koppelstück 30 ist der Ultraschallspiegel 31 angeordnet, der beispielsweise durch einen schräg angeschnittenen Metallzylinder mit einer als Nadelführung 32 dienenden Längsbohrung gebildet wird. Der emittierte Ultraschallstrahl wird an der Spiegelfläche 31a gegen die Austrittsfläche 225 umgelenkt, wobei die Strahlrichtung koaxial mit der Richtung der Nadelführung 32 verläuft. Der Raum 35 zwischen dem Spiegel 31 und der Austrittsfläche 225 ist beispielsweise mit Gelatine gefüllt, die den Ultraschall gut leitet.

Das Einschleusen der Ultraschallsonde 2 in die Kapsel des Sondenteils 20 wird im wesentlichen gleich wie beim ersten Ausführungsbeispiel durchgeführt.

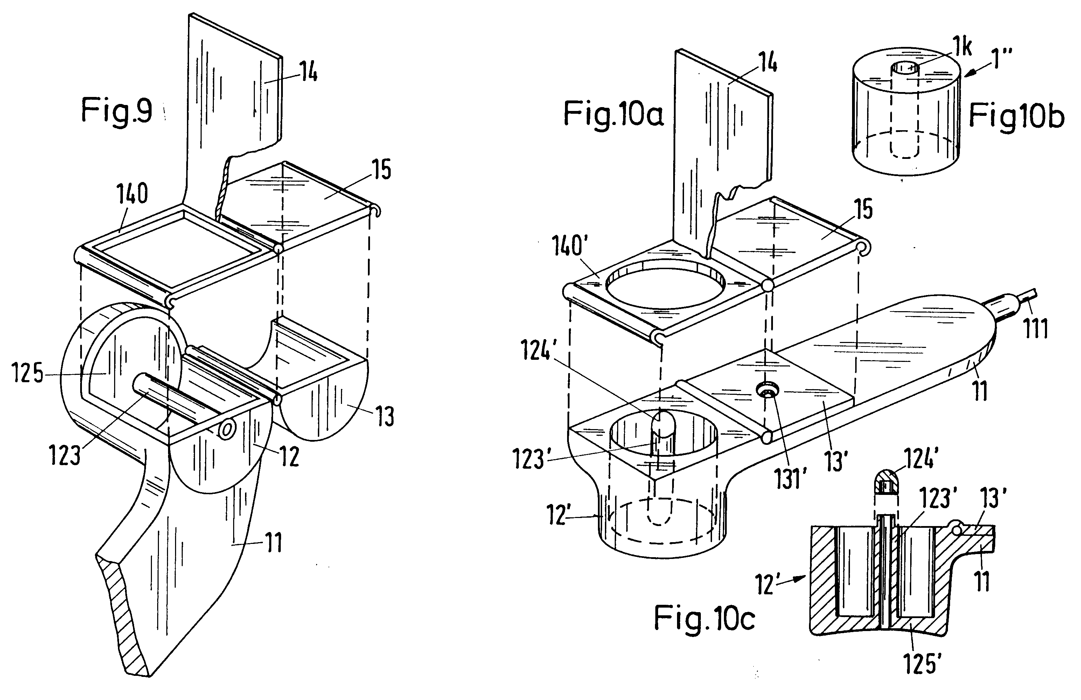

Eine variante Ausführungsform der Vorrichtung gemäss Fig.3 ist in Fig.9 dargestellt. Die Nadelführung 123 (statt 132) ist im Kapselteil 12 angeordnet, das mit dem Halter 11 verbunden ist. Die Positionen des Rahmens 140 und des inneren Deckels 15 sind gegenüber dem ersten Ausführungsbeispiel vertauscht. Beim Einschleusen der Ultraschallsonde 1 bzw 1′ gelangt somit diese in den Kapselteil 12. Bei der Schliessbewegung ist nun die Gefahr ausgeräumt, dass die Sonde 1 bzw. 1′ aus dem verschwenkten Kapselteil 13 gekippt werden könnte. Auf eine dem Schild 144 (Fig.2) entsprechende Abdeckung, die hier fehlt, kann verzichtet werden. Auch das zweite Ausführungsbeispiel (Fig. 6, 7) kann auf gleiche Weise abgewandelt werden, nämlich durch Vertauschen der Positionen des Deckels 25 bzw. des Rahmens 240, wobei ebenfalls der Schild 244 wegfällt.

Die Figuren 10a bis 10c zeigen ein erfindungsgemässes Punktiergerät, das eine zylindrische Sonde 1˝ mit einem axialen, seitlich geschlossenen Kanal 1k aufweist. Die Kapsel für die Sonde 1˝ wird hier durch ein zylindrisches, hülsenartiges Teil 12′ und einen ebenen Deckel 13′ gebildet. Das Kapselteil 12′, in Fig.10c als Längsschnitt dargestellt, enthält die Nadelführung 123′, auf die der Kanal 1k der Sonde 1˝ passt. Das oberer Ende der Nadelführung 123′ trägt eine Kappe 124′, die beim Einsetzen der Sonde 1˝ eine Verunreinigung der Nadelführung an ihrer oberen Eintrittstelle verhindert. Nach dem Einsetzen der Sonde wird die Kappe 124′ entfernt. Der Kapseldeckel 13′ weist eine Aussparung 131′ auf, die für das obere Ende der Nadelführung 123′ vorgesehen ist. Diese Aussparung 131′ist beispielsweise ein Sackloch, das durch eine durchstechbare Wand abgeschlossen wird; sie kann aber auch durch einen zur Nadelführung 123′ passenden Durchbruch gebildet sein. Der Kapseldeckel 13′ kann auf dem Halter 11 aufliegend, wie in Fig.10b dargestellt, angeordnet sein; er kann aber auch seitlich - also um 90° bezüglich der Achse der Nadelführung 123′ gedreht - oder auch auf der gegenüberliegenden Seite bzw. um 180° gedreht angeordnet sein. Die aus den beiden Deckeln 14 und 15 sowie dem Rahmen 140′ bestehende Einschleusvorrichtung ist im wesentlichen gleich wie bei den oben beschriebenen Ausführungsformen; lediglich der Rahmen 140′ zeigt statt einer rechteckigen nun eine kreisförmige Öffnung.

## Patentansprüche

1. Vorrichtung zum Vorbereiten eines sterilen Punktiergeräts für Blutgefässe, bei dem eine nichtsterile Ultraschallsonde (1) verwendbar ist, bestehend aus einem dichten Beutel (16) mit sterilem Inhalt, einem im Beutel (16) enthaltenen Halter (11) mit einer Kapsel (12, 13) für die Ultraschallsonde (1) und einer schleusenartigen Eintrittsstelle in der Beutelwand, über welche die Ultraschallsonde (1) in die Kapsel (12, 13) einschleusbar ist.

2. Vorrichtung nach Anspruch 1, dadurch gekennzeichnet, dass die schleusenartige Eintrittsstelle für den Durchtritt der Ultraschallsonde (1) eine Öffnung (162) mit einem Rahmen (140) in der Beutelwand aufweist, dass die Beutelöffnung (162) an der Aussenseite mit einem lösbaren, an einer Seite am Rahmen (140) befestigten Deckel (14) verschlossen ist und dass der Rahmen (140) auf der Innenseite mit einem zweiten Deckel (15) verbunden ist, mit welchem die Beutelöffnung (162) nach Einschleusen der Ultraschallsonde (1) verschliessbar ist.

3. Vorrichtung nach Anspruch 2, dadurch gekennzeichnet, dass der äussere Rand (165) der Beutelöffnung (162) und/oder der Rahmen (140) auf der Innenseite mit einem Haftmittel für den ersten bzw. den zweiten Deckel (14 bzw. 15) beschichtet sind.

4. Vorrichtung nach Anspruch 2, dadurch gekennzeichnet, dass auf der Innenseite des Rahmens (140) sowie am zweiten Deckel (15) Schnappverbindungselemente angeordnet sind.

5. Punktiergerät zu einer Vorrichtung nach einem der Ansprüche 1 bis 4, mit einer für die Ultraschallsonde (1) bestimmten Kapsel, die aus mindestens zwei aufklappbaren Schalenteilen (12, 13) besteht, wobei diese Schalenteile vor dem Einschleusen der Ultraschallsonde (1) mit der Eintrittsstelle des Beutels (16) verbunden sind.

6. Punktiergerät nach Anspruch 5 und zu einer Vorrichtung nach Anspruch 2, dadurch gekennzeichnet, dass die Kapsel aus zwei Schalenteilen (12, 13) besteht, wobei das eine mit dem Rahmen (140) und das andere mit dem zweiten Deckel (15) der Beutelöffnung (162) jeweils über ein Scharnier (141 bzw. 121) verbunden sind.

7. Punktiergerät nach Anspruch 6, dadurch gekennzeichnet, dass mindestens eines der Scharniere (121, 141) ein Filmscharnier ist.

8. Punktiergerät nach Anspruch 7, dadurch gekennzeichnet, dass das Filmscharnier (121, 141) Perforationen (122, 142) zum Trennen des Punktiergeräts von der Eintrittsstelle des Beutels (16) aufweist.

9. Punktiergerät nach Anspruch 6, dadurch gekennzeichnet, dass mindestens eines der Scharniere (121, 141) durch mindestens zwei schnappverbindungsartige Gelenke (225, 226) gebildet ist.

10. Punktiergerät nach einem der Ansprüche 5 bis 9, dadurch gekennzeichnet, dass die Kontaktflächen zwischen den Schalenteilen (12, 13), welche bei geschlossener Kapsel aufeinander zu liegen kommen, zumindest teilweise mit einem Haftmittel beschichtet oder mit Schnappverbindungselementen versehen sind.

11. Punktiergerät nach einem der Ansprüche 5 bis 10, dadurch gekennzeichnet, dass an der Kapsel ein Koppelstück (30) für den in der Sonde erzeugten sowie im Körper reflektierten Ultraschallstrahl angeordnet ist, in welchem ein Spiegel (31) für die Umlenkung des Ultraschalls und eine Führung (32) für eine Punktiernadel angeordnet sind.

12. Punktiergerät nach einem der Ansprüche 5 bis 10, dadurch gekennzeichnet, dass die Ultraschallsonde (1) aus zwei über ein Scharnier (1e) verbundenen Hälften besteht und dass eine Aussparung (1f, 1g) für einen Führungskanal (132) für eine Punktiernadel innerhalb der Kapsel, entlang der Längsachse der zusammengeklappten Ultraschallsonde (1), angeordnet ist.

13. Punktiergerät nach einem der Ansprüche 5 bis 10, dadurch gekennzeichnet, dass die Ultraschallsonde (1′) eine Nut (1i) aufweist, die für die Aufnahme einer Nadelführung (123) vorgesehen ist.

14. Punktiergerät nach einem der Ansprüche 5 bis 10, dadurch gekennzeichnet, dass die Ultraschallsonde (1˝) einen axialen, seitlich geschlossenen Kanal (1k) aufweist und die Kapsel für die Sonde (1˝) aus einem hülsenartigen Teil (12′) mit einer Nadelführung (123′) sowie einem Deckel (13′) besteht.
